# EUROPEAN PATENT APPLICATION

(11) **EP 4 579 678 A1**
(43) Date of publication of application: **02.07.2025**
(21) Application number: 23857783.7
(22) Date of filing: 25.08.2023
(51) Int. Cl.: G16H 30/20, G16H 50/50, G16H 50/70, G16H 50/20, G16H 50/30, G06V 10/72, G06V 10/764, G06V 10/46, G06V 10/56, G16H 70/60

(54) **TISSUE PATHOLOGY READING SUPPORT DEVICE AND METHOD THEREFOR**

(30) Priority: 25.08.2022 KR 20220106723
(71) Applicant: Seegene Medical Foundation, Seoul 04805 (KR)
(72) Inventor: CHUN, Jong Kee, Seoul 04805 (KR); KO, Young Sin, Seoul 04805 (KR); CHANG, Ji Wouk, Yongin-si Gyeonggi-do 16902 (KR)
(74) Representative: Bryn Aarflot AS
(86) International application number: PCT/KR2023/012646
(87) International publication number: WO 2024/043751

(57) **Abstract**

The present specification provides a tissue pathology reading support device and a method therefor, which, with respect to an artificial intelligence model, divide, into a plurality of patches, a slide tissue image for learning that indicates a lesion site if a lesion is present, infer the classification of each of the plurality of patches so as to learn patch classification results, integrate the plurality of patches and the patch classification results so as to infer the classification of the reconstructed slide tissue image, thereby learning slide tissue image classification results, use the trained artificial intelligence model so as to infer the classification of each of the plurality of patches, thereby generating patch classification results, and integrate the plurality of patches and the patch classification results so as to infer the classification of the reconstructed slide tissue image, thereby generating slide tissue image classification results.

## Description

### [Technical Field]

The present invention relates to a device and method for supporting histopathologic reading.

### [Background Art]

Digital pathology is a general term for all technologies that convert glass pathology slides into digital slides or slide tissue images (whole slide images (WSIs)) using a scanner and then observe them on a monitor via a personal computer or computer network instead of reading them using a conventional optical microscope.

The core technology of digital pathology is to quickly convert the entire shape of the tissues contained on a glass slide into a high-resolution digital image.

The ultimate goal of digitalizing histopathologic images is to make 'computer-assisted diagnosis' a reality by utilizing various automated image analysis computer algorithms.

Meanwhile, the workload of pathologists is steadily increasing, while the number of pathologists is relatively decreasing. The relative shortage of pathologists and their increasing workload may lead to a human error. Since the pathologic diagnosis of the histopathologic test actually means 'confirmative diagnosis', if the timing of early diagnosis and treatment is missed due to a false negative, there is no chance of recognition until the next test. Unfortunately, false negatives are the most common errors in pathologic diagnosis. Therefore, tools are needed to reduce or provide a chance of early detection and immediate correction of false negatives in repetitive, labor-intensive, and habitual reading work environments. Blind review is an important and effective method for enhancing quality control. However, it is not realistically possible to double check or review all the slides read every day.

A generally recommended quality control strategy for pathology laboratories is to randomly review a certain percentage of slides each month, but this percentage may vary depending on the circumstances of the institution. Sometimes significant inconsistencies arise during this process and are corrected at a later time. However, since the time is usually 1 to 2 months after the initial diagnosis report date, the follow-up measures are often too late to produce clinically meaningful results for patients. If artificial intelligence models could assist in that function, it would be a great help to both patients and pathologists.

If an artificial intelligence classification model is to be applied as a screening tool in the pre-reading stage, all target glass slides should be digitalized in advance.

As a predictable workflow, glass slides are created first, and digitalized and scanned, AI predictions are derived, and positive cases may be listed up first based on the prediction results. Thereafter, the specialist will open the slide tissue images (whole slide images (WSI)) through the viewer, make a diagnosis first through the scanned slides while referring to the predicted results or a heat map shown by the artificial intelligence, and if necessary, directly check the glass slides through a microscope, and otherwise, make a diagnosis only through the slide tissue images.

Digital pathology has developed rapidly and deeply in recent years and is already quickly transitioning into clinical practice, and has several advantages over traditional pathology. However, in the field of pathology, it is still difficult to fully replace the traditional glass slide-based reading. In particular, there is a concern that detection of microorganisms, such as Helicobacter pylori, in gastric biopsy tissue from slide tissue images is difficult. In pathology laboratories that routinely report classification and histologic grading of gastritis by the updated Sydney system, there is a practical burden to directly apply digital pathology to primary diagnosis without glass slides in gastric biopsy reading.

In particular, to directly apply slide tissue images to primary diagnosis, high-resolution (x40 or higher) scanning is required to minimize the difference from traditional under-a-microscope reading. In this case, significant costs are incurred in building and operating information computing infrastructure to store and process high-resolution slide tissue images. However, for quality control purposes rather than primary diagnostics, low-resolution (e.g., x20) scanned slide tissue images suffice.

In most commercial pathology laboratories, where reading is initiated immediately after slide production, the workflow change to postpone the reading step until after slide scanning may be a significant burden. Because of these limitations, some researchers have proposed augmented reality microscope (ARM) with real-time artificial intelligence integration instead of slide tissue image-based ones. However, no matter how high-performance artificial intelligence-applied (ARM) it is, it cannot detect lesions outside the field of vision of a microscope, so it still has limitations in compensating for routine human errors made by pathologists. Further, there is no published or commercialized model of ARM scheme applicable to endoscopic biopsy reading.

Lastly, when artificial intelligence models prioritize determines, visualize suspicious lesions, and provide expected classification results before reading by pathologists, the pathologists may be subjected to AI-dependent bias. Although several artificial intelligence models have been introduced that show performance comparable to the diagnostic capabilities of pathologists, the legal responsibility and authority for the diagnostic results of each case ultimately lies with the pathologist. Bias is a problem in itself, and may even cause dependency as the performance of AI models enhances. This may be a concern for patients as well. When a pathologist who relies on a high-performance artificial intelligence model makes a diagnosis with bias, which may lead to a misdiagnosis (e.g., when both the artificial intelligence model and the human pathologist miss a lesion, or when the human pathologist reads while ignoring the slide tissue image by believing the artificial intelligence model's prediction of a negative result), there is a possibility that a legal dispute will arise over whether there is liability.

In recent years, many researchers have developed and proposed artificial intelligence models based on histopathology slide images, and in fact, some studies claim that they show performance similar to or superior to that of pathologists. However, ultimately, the full responsibility and authority for each diagnosis lies solely with the pathologists involved, which cannot be fully replaced by artificial intelligence.

In particular, in gastrointestinal pathology, carcinoma (CA)/high-grade dysplasia (HGD) screening has inter-observer variability or inconsistency depending on the group. In gastrointestinal pathology, it is easy to predict that the performance of each developed artificial intelligence classification will inevitably vary depending on how the classifications are defined. In fact, other similar studies have shown differences in the definitions of classifications according to groups, and some studies have excluded diagnoses that fall within the gray area.

Further, in many cases, pathologists use ambiguous language in reports of endoscopic biopsies (or other small biopsies) specimens, unlike resection specimen reading, in their daily practice. Some examples are atypical glandular proliferation of undetermined significance, suspicious for dysplasia, cannot be ruled out malignancy, favor neoplastic... In this reality, the expectation that artificial intelligence models will address these concerns of pathologists is close to an illusion. This is because of the limitations of small biopsy tissue reading itself.

However, many studies focus on enhancing the performance of artificial intelligence models in accurately diagnosing the legion of interest (region of interest (ROI)), which often leads to low reproducibility in clinical practice. This is because the conditions for high accuracy of artificial intelligence models are very different from the conditions in the reality of daily practice. To ensure high accuracy of artificial intelligence models, preparation for well-refined data is important. In fact, poor scan quality (out of focus, tissue missing, air bubbles.. etc.)) and poor slide quality (poor staining, poor section, tissue artifact, air bubbles, tissue folding, poor dehydration, etc.) are factors that reduce the performance of the model. When the model is trained only with very refined data with all of the artifacts excluded and scanned cleanly without blur from really well-made slides, the model's performance will be maximized. However, given the switch to a 'fully digitalized pathology laboratory', it may be quite contradictory and unrealistic.

As mentioned earlier, the number of histopathologic tests is gradually increasing, which is not only increasing the workload of pathologists, but also putting an overload on pathology laboratory technicians who prepare the slides. The increased workload also affects the work capacity of histopathology technicians, which leads to a decline in the quality of slides. In other words, pathologists cannot control every slide to perfect quality every day, and in reality, they encounter a higher percentage of slides that are of substandard quality than expected. The same is also true for the quality of the scan. The businesses report scan error rates of around 1-3%. These slides may be of such poor quality that they cannot be fully read, or slide tissue images that are of such poor quality that they may be read to some extent but exhibit the various artifacts mentioned above are frequently seen. It is not realistic and inefficient to rescan all low-quality slide tissue images although readable. In terms of developing usable artificial intelligence models and applying them in practice," it would be more appropriate to develop and apply reliable artificial intelligence models that reflect reality in line with routine practice, rather than taking on this inefficient workload just to maximize artificial intelligence model performance.

### [Detailed Description of the Invention]

### [Technical Problem]

Embodiments provide devices and methods for supporting reading of tissue pathology, which reduce potential human errors, particularly false negatives, that pathologists routinely experience.

### [Technical Solution]

The disclosure provides a histopathologic reading support device and method that, when there is a legion, trains an artificial intelligence model with patch classification results by dividing a training slide tissue image marked with a site of the legion into a plurality of patches and inferring a classification of each of the plurality of patches and trains the artificial intelligence model with a slide tissue image classification result by inferring a classification of a slide tissue image reconstructed by integrating the plurality of patches and the classification results of the patches; a tissue classification step generating a patch classification result by inferring a classification of each of the plurality of patches using a trained artificial intelligence model, and generates a slide tissue image classification result by inferring a classification of a slide tissue image reconstructed by integrating the plurality of patches and the classification results of the patches.

An embodiment provides a histopathologic reading support device comprising an input unit inputting slide information and a slide tissue image, a pre-processing unit dividing the input slide tissue image into a plurality of patches, a tissue classification unit generating a patch classification result by inferring a classification of each of the plurality of patches using a trained artificial intelligence model, and generating a slide tissue classification result by inferring a classification of a slide tissue image reconstructed by integrating the plurality of patches and the classification results of the patches, a storage unit storing the slide information and the slide tissue image, the plurality of patches, patch information about the patches, the patch classification result, and the slide tissue image classification result, and an output unit outputting the patch classification result of each of the plurality of patches and the slide tissue image classification result.

When there is a legion, the trained artificial intelligence model may be trained with patch classification results by dividing a training slide tissue image marked with a site of the legion into a plurality of patches using the pre-processing unit and inferring a classification of each of the plurality of patches and is trained with a slide tissue image classification result by inferring a classification of a slide tissue image reconstructed by integrating the plurality of patches and the classification results of the patches.

Another embodiment provides a histopathologic reading support method, comprising an input step inputting slide information and a slide tissue image, a pre-processing step dividing the input slide tissue image into a plurality of patches, when there is a legion, a training step training an artificial intelligence model with patch classification results by dividing a training slide tissue image marked with a site of the legion into a plurality of patches and inferring a classification of each of the plurality of patches and training the artificial intelligence model with a slide tissue image classification result by inferring a classification of a slide tissue image reconstructed by integrating the plurality of patches and the classification results of the patches, a tissue classification step generating a patch classification result by inferring a classification of each of the plurality of patches using a trained artificial intelligence model, and generating a slide tissue image classification result by inferring a classification of a slide tissue image reconstructed by integrating the plurality of patches and the classification results of the patches, a storage step storing the slide information and the slide tissue image, the plurality of patches, patch information about the patches, the patch classification result, and the slide tissue image classification result, and an output step outputting the patch classification result of each of the plurality of patches and the slide tissue image classification result.

### [Advantageous Effects]

By the device and method for supporting reading of tissue pathology according to embodiments, it is possible to reduce potential human errors, especially false negatives, that pathologists routinely experience.

### [Brief Description of the Drawings]

FIG. 1 is a concept view illustrating a histopathologic reading support system according to an embodiment;
FIG. 2 is a block diagram illustrating a histopathologic reading support device according to an embodiment;
FIG. 3 illustrates processes of training the artificial intelligence model of FIG. 2;
FIG. 4 is a flowchart illustrating a data pre-processing process for generating a patch image in the data pre-processing process of FIG. 3;
FIG. 5 illustrates an operation flow and the results of the patch classification unit of FIG. 3;
FIG. 6 illustrates an operation flow and the results of the slide tissue image classification unit of FIG. 3;
FIG. 7 illustrates an operation flow and the results of the tissue classification unit of FIG. 2;
FIG. 8 is a flowchart illustrating slide analysis results and slide tissue image classification results through the histopathologic reading support device of FIG. 2;
FIG. 9 illustrates a slide tissue image displayed on the display unit of FIG. 2;
FIG. 10 illustrates an entire reading result, a slide preview image, an artificial intelligence reading result, and previous result information displayed on the display unit of FIG. 2;
FIG. 11 illustrates whether there is a discordance between the slide tissue image classification result and statistical information and slide analysis result displayed on the display unit of FIG. 2;
FIG. 12 is a flowchart illustrating a histopathologic reading support method according to another embodiment; and
FIG. 13 is a block diagram illustrating a computing system according to embodiments of the present invention.

### [Mode for Carrying out the Invention]

Hereinafter, exemplary embodiments of the inventive concept will be described in detail with reference to the accompanying drawings. The inventive concept, however, may be modified in various different ways, and should not be construed as limited to the embodiments set forth herein. Like reference denotations may be used to refer to the same or similar elements throughout the specification and the drawings. However, the present invention may be implemented in other various forms and is not limited to the embodiments set forth herein. For clarity of the disclosure, irrelevant parts are removed from the drawings, and similar reference denotations are used to refer to similar elements throughout the specification.

In embodiments of the present invention, when an element is "connected" with another element, the element may be "directly connected" with the other element, or the element may be "electrically connected" with the other element via an intervening element. When an element "comprises" or "includes" another element, the element may further include, but rather than excluding, the other element, and the terms "comprise" and "include" should be appreciated as not excluding the possibility of presence or adding one or more features, numbers, steps, operations, elements, parts, or combinations thereof.

When the measurement of an element is modified by the term "about" or "substantially," if a production or material tolerance is provided for the element, the term "about" or "substantially" is used to indicate that the element has the same or a close value to the measurement and is used for a better understanding of the present invention or for preventing any unscrupulous infringement of the disclosure where the exact or absolute numbers are mentioned. As used herein, "step of" A or "step A-ing" does not necessarily mean that the step is one for A.

As used herein, the term "part" may mean a unit or device implemented in hardware, software, or a combination thereof. One unit may be implemented with two or more hardware devices or components, or two or more units may be implemented in a single hardware device or component.

As used herein, some of the operations or functions described to be performed by a terminal or device may be, instead of the terminal or device, performed by a server connected with the terminal or device. Likewise, some of the operations or functions described to be performed by a server may be performed by a terminal or device connected with the server, instead of the server.

In the disclosure, the training process may be expressed as training, and the result may be expressed as learning but the training process or the result may be referred to as one of training or learning.

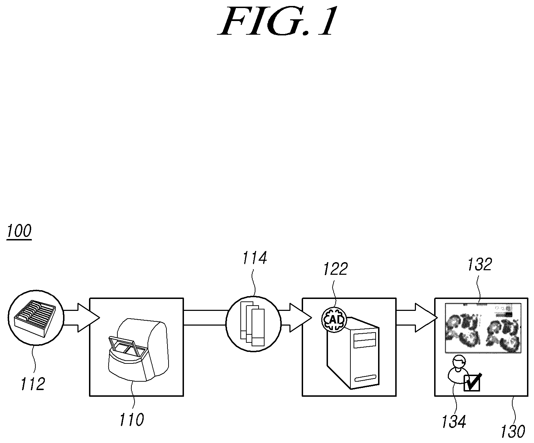

FIG. 1 is a conceptual view illustrating an automatic verification system for diagnosis in routine practice of histopathologic reading according to an embodiment.

Referring to FIG. 1, an automatic verification system 100 for diagnosis in histopathologic reading routine practice according to an embodiment includes a slide scanner 110, a server computer 120, and a display device 130.

If an operator inputs a tissue slide 112, the slide scanner 110 generates a digitalized slide tissue image 114 and, if it is uploaded to the server computer 120, a developed artificial intelligence model 122 generates an output on the display device 130.

The output may be, e.g., lesion location information 132 including the locations of the tissue lesions and patient reading information 134 including the classification results in the units of patients. In other words, the display device 130 displays the lesion location information 132 including the locations of the tissue lesions and patient reading information 134 including the classification results in the units of patients.

Further, the output additionally displays whether the slide tissue classification results diagnosed by the histopathology support device using the artificial intelligence model and the pathologic diagnosis, which is the slide reading result by the pathologist, discord, constructing the automatic verification system 100 for diagnosis in the routine practice of histopathologic reading by the artificial intelligence model. Accordingly, it is possible to manage the diagnostic quality of routine practice during pathologic diagnosis.

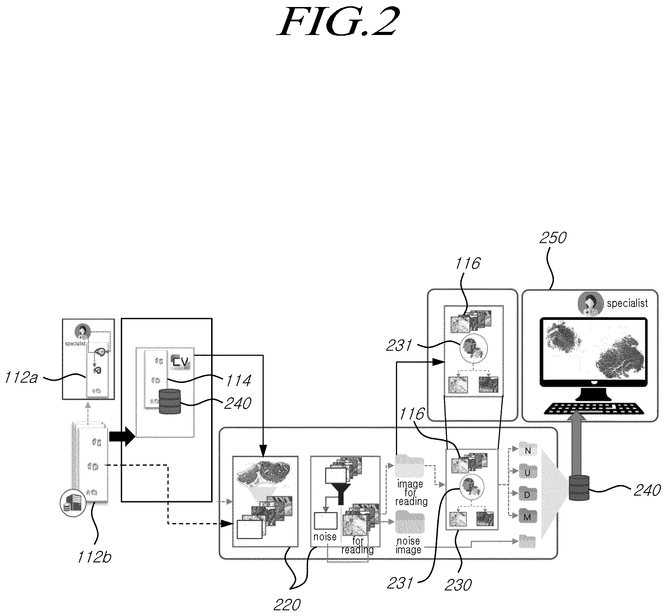

The above-described server computer 120 may include the histopathologic reading support device 200 described with reference to FIGS. 2 to 10 and 11. Hereinafter, the histopathologic reading support device 200 is described in detail.

FIG. 2 is a block diagram illustrating a histopathologic reading support device according to an embodiment.

Referring to FIG. 2, a histopathologic reading support device 200 according to an embodiment may include an input unit 210, a pre-processing unit 220, a tissue classification unit 230, a storage unit 240, and an output unit 250.

The input unit 210 inputs slide information, which is information about the tissue slide, and a slide tissue image obtained by digitalizing the tissue slide.

As described with reference to FIG. 1, if the tissue slide 112 is input by the operator, the slide scanner 110 generates a digitalized slide tissue image 114. The slide tissue image 114 may be a training slide tissue image and a reading slide tissue image. The training slide tissue image is a digitalized slide tissue image of the learning slide 112a, and the reading slide tissue image is a digitalized slide of the reading slide 112b.

The input unit 210 may receive the slide tissue image obtained by capturing the tissue from the outside of the histopathologic reading support device 200. The slide tissue image is an image in which the tissue is captured.

The tissue may contain human or animal cells. For example, the tissue may be a biopsy tissue extracted from an organ (e.g. stomach, colon, small intestine, and liver) of a human body to identify the presence of cancer (e.g. stomach cancer or colon cancel) cells.

The tissue may be captured through various types of capturing devices (e.g. cameras, camcorders, or scanners). Further, the format of the image in which the tissue was captured may be variously determined as JPG, GIF, PNG, BMP, etc.

In this case, the tissue may be a tissue obtained by staining with hematoxylin and eosin. By hematoxylin and eosin staining, the cell nuclei may be stained blue, and the extracellular matrix and cytoplasm may be stained pink.

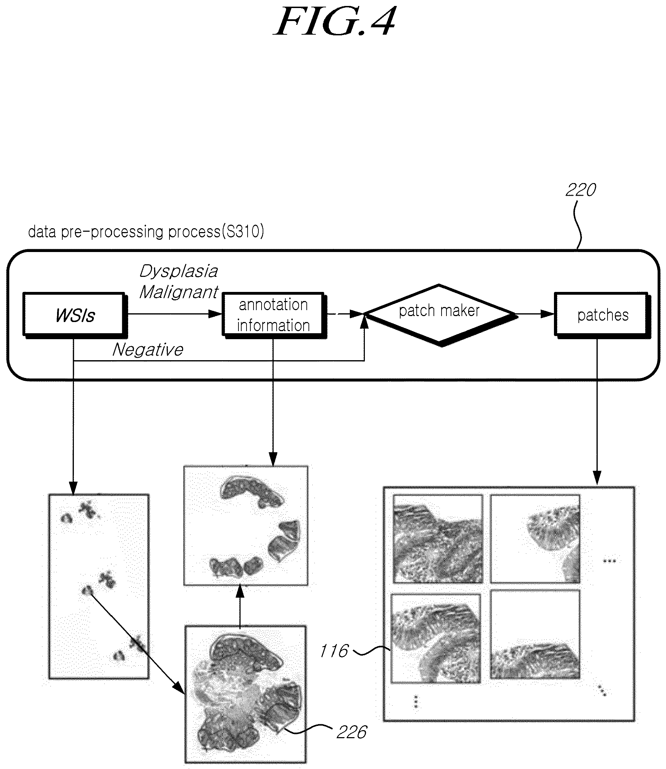

Meanwhile, the training slide tissue image may further include annotation information. The annotation information is information indicating which area of the tissue image corresponds to which group. For example, the annotation information may indicate that a specific area of the tissue image is an abnormal area in which the lesion (e.g. cancer) exists and that the remaining area is a normal area in which no lesion exists. For example, as described below with reference to FIG. 4, the training slide tissue image may, if a lesion exists, indicate the lesion site by a single closed curve in a different color for each lesion, as the annotation information.

The annotation information may be used as information indicating a correct answer when training the artificial intelligence model 231 for classifying the slide tissue image.

The pre-processing unit 220 may divide the slide tissue image 114 input from the input unit 210 into a plurality of patches 116.

The size of each of the plurality of patches 116 may be smaller than the tissue image. For example, the respective sizes of the plurality of patches 116 may be the same as n*n pixels (e.g. 256*256 or 128*128).

As another example, the sizes of the plurality of patches 116 may be different from each other. Some of the plurality of patches 116 may be patches having a size of n*n pixels (e.g., 256×256), and some may be patches having a size of m*m pixels (e.g., 128×128). At this time, m is smaller than n.

The tissue classification unit 230 generates a patch classification result by inferring a classification of each 116 of the plurality of patches using a trained artificial intelligence model 231, and generates a slide tissue classification result by inferring a classification of a slide tissue image reconstructed by integrating the plurality of patches 116 and the classification results of the patches.

When there is a legion, the trained artificial intelligence model 231 may be trained with patch classification results by dividing a training slide tissue image 114 marked with a site of the legion into a plurality of patches 116 using the pre-processing unit 220 and inferring a classification of each of the plurality of patches 116 and is trained with a slide tissue image classification result by inferring a classification of a slide tissue image 114 reconstructed by integrating the plurality of patches 116 and the classification results of the patches.

The above-described artificial intelligence model 231 may be a deep learning model. In the disclosure, the deep learning model may be a model in which artificial neural networks are stacked in multiple layers. The deep learning model may be implemented as a model that trains the network in a manner to automatically the features of each image by learning massive data in a deep neural network composed of a multi-layered network and minimize errors, in the target function, i.e., prediction accuracy, therethrough.

For example, the deep learning model may be a convolutional neural network (CNN). The CNN may be implemented in various schemas, such as visual geometry group (VGG) network, inception (GoogleNet), ResNet, and DenseNet.

However, the deep learning model described in embodiments of the present invention is not limited to the CNN, but other types of deep learning models (e.g., deep hierarchical network (DHN), convolutional deep belief network (CDBN), deconvolutional deep network (DDN), or recurrent neural network (RNN)) which may be used currently or in the future may be used.

The deep learning model may be implemented through a deep learning framework. The deep learning framework plays a role to provide a library of functions commonly used when developing the deep learning model and to support the system software or hardware platform to be properly used. In this embodiment, the deep learning model may be implemented using any deep learning framework that has been currently disclosed or will be disclosed in the future.

Training the deep learning model means adjusting the parameters for the deep learning model so that the prediction value for the input of the deep learning model is as similar to the actual value (ground-truth) as possible. The tissue classification unit 230 may increase the prediction accuracy of the deep learning model by repeatedly performing the process of inputting the patch 116 and the reconstructed slide tissue image (reconstructed WSI) to the deep learning model 231, and adjusting parameters for the deep learning model 231 by comparing the prediction value and the actual value of the deep learning model.

In this case, the tissue classification unit 230 may adjust the parameters for the deep learning model 231 in a way to minimize the loss function values of the input patch 116 and the reconstructed slide tissue image.

The loss function is a function that calculates how similar the prediction value of the type for the target patch is to the actual type of the target patch. The loss function may be, e.g., mean absolute error (MAE), mean square error (MSE), root mean square error (RMSE), binary cross-entropy, categorical cross-entropy, or the like.

There are two or more trained artificial intelligence models 231, and the slide information may include an organ of the slide tissue image. In this case, the tissue classification unit 230 may classify the tissue image by selecting one of the two or more artificial intelligence models according to the organ of the slide tissue image. As an example of the trained artificial intelligence model 231, an artificial intelligence model trained with organs of the stomach tract and colon is described below. However, in addition to the illustrated stomach and colon models, models for other organs (breasts, prostate, skin, etc.) may be further developed in a similar way and applied as a routine quality control system in the same way.

In embodiments of the present invention, the patch classification results and the slide tissue classification results may be classified in various ways.

For example, the patch classification results and the slide tissue classification results may be classified into two groups or classes, such as malignant and non-malignant, or neoplastic and non-neoplastic.

As another example, the patch classification results and the slide tissue classification results may be classified into three groups: malignant, dysplasia, and non-neoplastic.

As another example, the patch classification results and the slide tissue classification results may be classified into four groups: malignant, dysplasia, uncategorized, and non-neoplastic.

For example, the malignant group may be defined as a group indicating malignant neoplasms, including adenocarcinoma, suspicious for adenocarcinoma, suggestive of adenocarcinoma, high-grade lymphoma, and other carcinomas.

The dysplasia group may be defined as a group indicating dysplasia including tubular adenoma with dysplasia of all grades.

The non-neoplastic type may be defined as a group indicating non-neoplastic benign lesions (e.g. gastritis, polyps, etc.).

The uncategorized group may be defined as a type indicating the remaining lesions that do not correspond to the three groups described above, such as atypical glandular proliferation, neuroendocrine tumors, submucosal tumors, low-grade lymphoma, and stromal tumors.

In this case, some of the patch classification results and the slide tissue classification results may be defined as a normal group, and the rest except for the normal group may be defined as an abnormal group. For example, if the patch classification results and slide tissue classification results are classified into four groups: malignant, dysplasia, uncategorized, and non-neoplastic, malignant, dysplasia, and uncategorized may be defined as the abnormal group, and non-neoplastic may be defined as the normal group.

The storage unit 240 may store the slide information and the slide tissue image, the plurality of patches, patch information about the patches, the patch classification result, and the slide tissue classification result. The storage unit 240 may temporarily store some of the above pieces of information and then automatically delete them, or store them continuously until a delete request is made.

The output unit 250 may output the patch classification result of each of the plurality of patches 116 and the slide tissue classification result. The output unit 250 may output not only the patch classification result of each of the plurality of patches 116 and the slide tissue classification result, but also all information input through the input unit 210 and all information stored in the storage unit 240.

In line with the development intention to develop an tissue classification model using the artificial intelligence model 231 with the aim of using it for quality control of routine practice in real life, the principle in data preparation is to use the data, as it is, reserving inter-observant variability and routine artifacts in real life, to thoroughly reflect the situations in the practice rather than artificially preparing a well-refined slide tissue image data set to secure the high accuracy of the model 231.

The slide quality may not be manipulated for the purpose of ensuring the best model accuracy. Rather, common artifacts - slide cuts, poor staining, poor section, air bubbles, etc. - may be used for training as they are without artificial enhancement (e.g., re-preparing high-quality slides dedicated to training). However, poor slides difficult for pathologists to read may be excluded.

In other words, the training slide tissue image may use the slide tissue image scanned by the slide scanner as it is without artificial enhancement.

The scan quality may also not be artificially adjusted. According to the scanner manufacturer, the defect rate requiring re-scanning, of 1-3% on average, is notified of, and most of the others are insignificant issues that do not affect reading. Examples are focus-out in a narrow range, overlap of some tiles, or edge cutting in some scanned tissue. To reflect these common minor issues as they are, artificial enhancements may not be made, such as re-scanning to obtain high-quality slide tissue images dedicated to training. However, as illustrated in FIG. 2, a scan failure at a level that is difficult for the pathologist to read due to a noise image may be excluded.

The patch classification results may be defined into four groups, as follows, as a four-classification model to classify all possible diagnoses (Table 1). However, by definition, 'group U' with the greatest heterogeneity of diseases within the group may be a factor that deteriorates the model's performance a lot, so that a three-class model excluding 'group U' may be adopted. Cases corresponding to group U may be finally excluded from the training data for developing a three-classification model. However, as a next step, it may be redefined to include only NET (grade 1 or 2) of group U in Table 1 and upgraded to a four-classification model for practical application.

**[Table 1]**

| Group | Definition | Color |
|---|---|---|
| M*(Malignant)* | Diagnosed as malignant neoplasm, including adenocarcinoma, suspicious for (s/f) adenocarcinoma, suggestive of (s/o) adenocarcinoma, (s/f, s/o) high-grade lymphoma, and any other (s/f, s/o) carcinoma or malignant neoplasm. | Red |
| D*(Dysplasia)* | Diagnosed as dysplasia, including (s/f, s/o, favor) adenoma with dysplasia of any grade. | Blue |
| N*(Negative for dysplasia)* | Diagnosed as non-neoplastic mucosal lesion, including inflammation, ulcer and polyps. | - |
| | | |

The sliding tissue images corresponding to each classification may be independently annotated by each pathologist. Group M is drawn in a single red closed curve, group D is drawn in a single blue closed curve, and when the boundary between the legion portion and the normal portion is unclear, an outline may be drawn to include only obvious legions (including both the epithelium and the stroma). Only patches within the single closed curve are used as data for each classification, and all of the other portions may be removed.

For group N, slide tissue images that meet the above definition may be reviewed and confirmed by pathologists and patches are immediately generated without additional annotation and used as data. In this case, portions other than the lesions of the group M and D slides may not be used as the group N data. It is possible to avoid including non-neoplastic glands or crypts as much as possible within one closed curve, and to avoid including non-tissue components such as necrosis, ulcer detritus, extracellular mucin pool, and blood as much as possible. If necessary, annotations may be reviewed by other pathologists and, in some cases, modified or excluded.

The slide tissue image 114 is input to the artificial intelligence model 231, and the artificial intelligence model 231 is required to classify a group for the slide tissue image and predict the result. The slide tissue image 114 was converted and processed into a form recognizable by the artificial intelligence model 231, and the artificial intelligence model is constructed to process without information loss in 1) the training step and 2) the prediction step.

Since the slide tissue image 114 includes giga pixel-level information, it is not suitable to use it in the deep learning model at once. Accordingly, the slide tissue image 114 including the corresponding to giga-pixel information is converted to be processed by the artificial intelligence model 231.

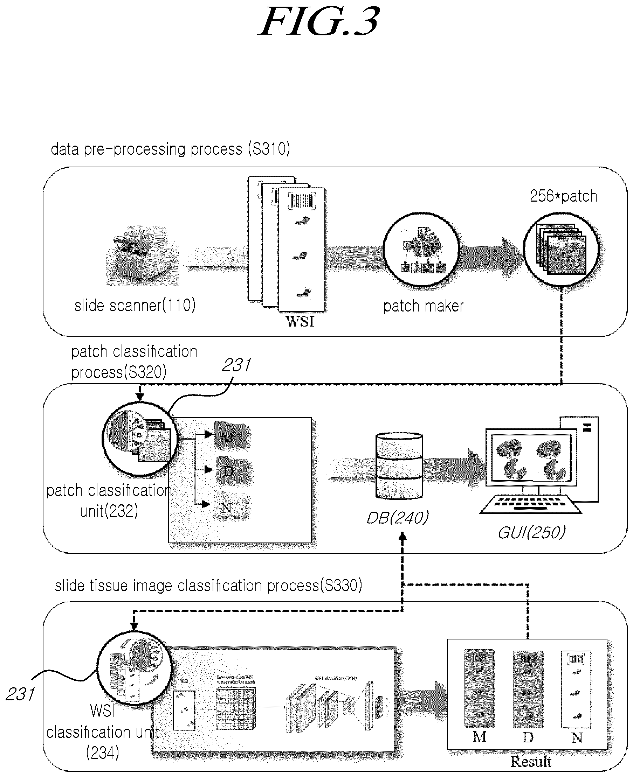

A model is constructed based on a method of converting the slide tissue image 114 into a patch form and processing it. As mentioned in many convolutional neural network (CNN)-based slide tissue image processing studies, processing complete slide tissue images at once may cause loss of information contained in the slide tissue images and resource loss. Therefore, the model is trained according to the order of operation as shown in FIG. 3.

The output unit 250 may indicate the patch classification result in a unique color for each legion position of each patch, and indicate the slide tissue classification result with at least one of a letter, a number, or a color.

The reconstructed slide tissue image is reconstructed by combining the plurality of patches displayed in a unique color for each location of the lesion, and the output unit 250 may display the slide tissue analysis results indicated by letters or numbers together with the reconstructed slide tissue image.

The input unit 210 may input the pathologic diagnosis which is the slide reading result of examining the slide corresponding to the slide tissue image through a microscope or the slide tissue image through a screen by the pathologist, and the display unit 250 may additionally display whether the slide tissue image classification result by the tissue classification unit and the pathologic diagnosis which is the slide reading result by the pathologist discord together with the pathologic diagnosis which is the slide reading result by the pathologist. Through this, it is possible to manage the diagnostic quality in the routine practice by constructing an automatic verification system for diagnosis in the routine practice of histopathologic reading with the artificial intelligence model 231.

Specifically, what is input to the input unit 210 may be content read by the pathologist, i.e., "pathologic diagnosis" information (text information). This pathologic diagnosis information may be "classified" as one of three classes M, D, and N by a classification rule. The artificial intelligence model may independently predict one of the three classes M, D, and N. The display unit 250 displays whether "classification result by pathologic diagnosis information (text information)" and "classification result by artificial intelligence model (image information)" corresponding thereto accord or discord. When the display unit 250 additionally displays whether the slide tissue image classification result and the pathologic diagnosis which is the slide reading result by the pathologist discord for the above-described two or more cases, if one of the slide tissue image classification result and the pathologic diagnosis which is the slide analysis result by the pathologist is positive, the display unit 250 may display it in priority.

FIG. 3 illustrates processes of training the artificial intelligence model of FIG. 2. FIG. 4 is a flowchart illustrating a data pre-processing process for generating a patch image in the data pre-processing process of FIG. 3.

Referring to FIGS. 3 and 4, in order to train the artificial intelligence model used by the tissue classification unit 230, a process S310 of pre-processing data, a process S320 of classifying patches, and a process S330 of classifying the slide tissue image are included.

In the process of pre-processing data (S310), the data is organized by the pre-processing unit 220, and the artificial intelligence model is converted into several patches in a trainable form.

The data pre-processing process S310 trains the model 231 based on labeled information as shown in Table 1 and the actual value information generated as by the above-described method. The training slide tissue image (WSI) of group N may set up data as it is, and for groups M and D, data may be set up including, e.g., annotation information indicating the lesion site in color.

Two deep neural networks (DNNs) models are largely needed for learning slide tissue images (WSIs) for training. One model may be used for the patch classification unit 232 and the other may be used for the slide tissue image classification unit 234. Therefore, for the training of each model, two types of data: 'patch image data' and 'slide tissue image data' are prepared.

First, for the slide tissue image data, the scan configuration values of the collected slide tissue image (WSI) are identified, and slide tissue images with different values are excluded from the data set. This is to prevent the slide tissue images (WSI) taken under different conditions from causing errors in the model. The finally selected slide tissue image data set is shown in Table 1.

For group N, square patches with a size of 256*256 pixels are generated from the training slide tissue image (WSI). The patch size may be defined by investigating the trade-off between user convenience and performance by an interview. For example, it may be identified that patches with large pixel sizes may have good performance, but are not appropriate for providing a description of legion location information provided from the user interface.

Next, for groups M and D, only patches present in the annotation with respect to the annotation information may be selected and stored. Patches outside the patch may not be included in the data set.

Further, for accurate model performance evaluation and feedback, training, evaluation, and inspection sets are configured based on slide tissue images (WSIs) to which patch data belongs. If the training, evaluation, and inspection sets are constructed without consideration of the relevance between the slide tissue images (WSI) and patches, patch images generated from the same slide tissue image (WSI) in the configuration of the training set are distributed simultaneously in the training data set and the evaluation and inspection data sets, so that the model may operate as cheating in the inspection step, making accurate evaluation difficult. Finally, in order to minimize the bias generation of the tissue classification unit 230, patch data may be randomly sampled from the generated training pool.

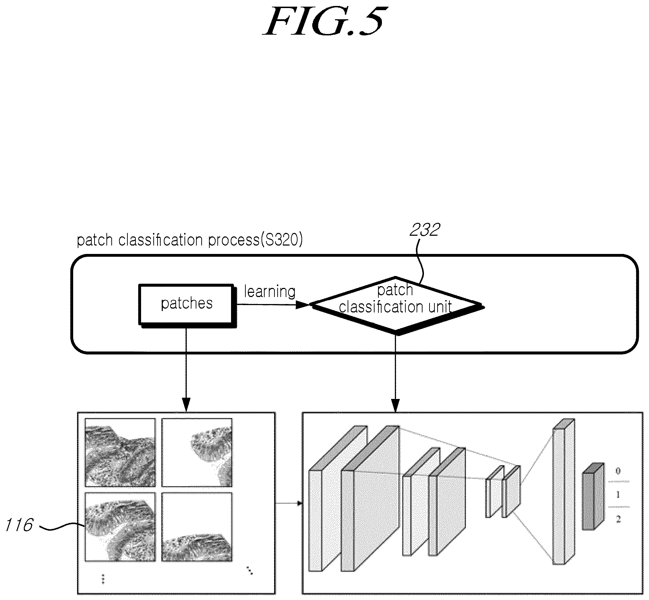

FIG. 5 illustrates an operation flow and the results of the patch classification unit of FIG. 3.

Referring to FIGS. 3 and 5, the patch classification process S320 performs training with respect to labeling information on each patch generated from one training slide tissue image (WSI) by the patch classification unit 232.

The patch classification process S320 trains the patch classification unit 232 using a CNN-based DNNs architecture. For example, the model may be trained by utilizing DenseNet201, which shows high performance among DNN architectures for classification of patch images. The set-up training data may be put into the trained DenseNet201 model. The patch classification model may train each patch image to infer three correct answer labels, e.g., groups M, D, and N.

The patch classification result generated by the patch classification unit 232 aims at two usages: 1) use as elements for inferring by the slide tissue image classification unit 234; and 2) providing a description in the user interface. In particular, for use as elements for the slide tissue image classification unit 234, the slide tissue image (WSI) may be learned based on the model trained in this step. The patch classification unit 232 may generate the patch classification result in the form of a distribution for each group, which is used as key information.

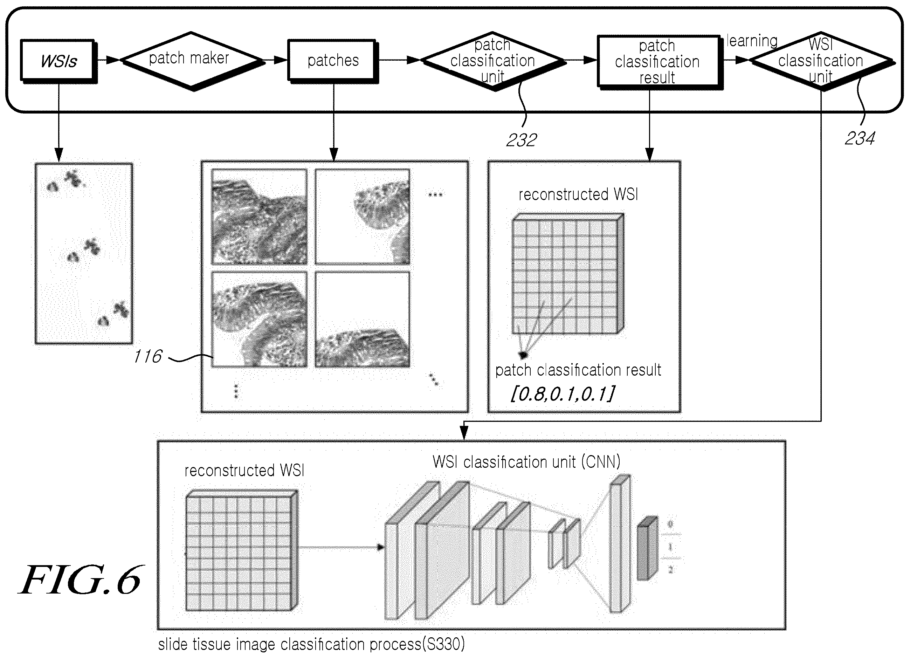

FIG. 6 illustrates an operation flow and the results of the slide tissue image classification unit of FIG. 3.

Referring to FIGS. 3 and 6, the slide tissue image classification process S330 trains an artificial intelligence model that generates patch classification information for the slide tissue image (WSI) by integrating information about the slide tissue image (WSI) by the training slide tissue image classification unit 234. The training method of the artificial intelligence model is applied equally to each artificial intelligence model for two or more organs to construct the model.

The slide tissue image classification unit 234 performs training to infer the training data which uses the patch classification results which are the fragmentized information of the slice as elements, with the correct answer table of the slide tissue image (WSI).

The training step of the slide tissue image classification unit 234 trains the slide tissue image classification unit 234 through a series of processes based on 1) the patch maker model used in the data pre-processing step and 2) the patch classification unit 232 trained in the model training. This is to efficiently utilize the resources necessary for classifying slide tissue images containing giga-pixel information, and each model is designed to operate organically like one model.

In the training process of the slide tissue image classification unit 234, the slide tissue image (WSI) is converted into a patch image by a patch maker, and the converted patch image is reconstructed based on location information and is learned by the slide tissue image classification unit 234.

The training process of the slide tissue image classification unit 234 largely includes three steps; a patch maker step, a patch classification step, and a slide tissue image learning step.

In the patch maker step, the slide tissue image to be learned is converted into several patch images. In order to preserve the location information about each patch image in the corresponding conversion process, the index and location information about the converted patch and which slide tissue image it was generated are recorded in the storage unit 240.

Next, in the patch classification step, through the trained patch classification unit 232, what classification information each patch constituting one slide tissue image has is inferred.

The inference information about each patch image is stored in the storage unit 240 according to the index of the patch. Therefore, the index, location information, and classification information about each patch image with respect to one slide tissue image are comprehensively recorded in the storage unit 240. Finally, a reconstructed slide tissue image (WSI) is generated by integrating the storage unit 240 of the patch image for learning the slide tissue image. Since the reconstructed slide tissue image includes feature information about the slide tissue image in a summarized or compressed form, the corresponding information may be processed in the CNN as one image. Accordingly, a CNN group classification network having the reconstructed slide tissue image in an input form is constructed, and the model is trained to appropriately infer three correct answer labels, e.g., groups M, D, and N.

Referring to FIG. 6, a process is shown in which for one slide tissue image, the patch is converted into multiple images by the patch maker, and distribution information is generated by the patch classification unit 232, and this is converted into a reconstructed slide tissue image.

It may be identified that the reconstructed slide tissue image is configured in a shape as a general square image. The CNN model trained to classify the reconstructed slide tissue image is used as the slide tissue image classification unit 234. The above series of processes are performed on each of the gastric biopsy and colon biopsy, generating two models.

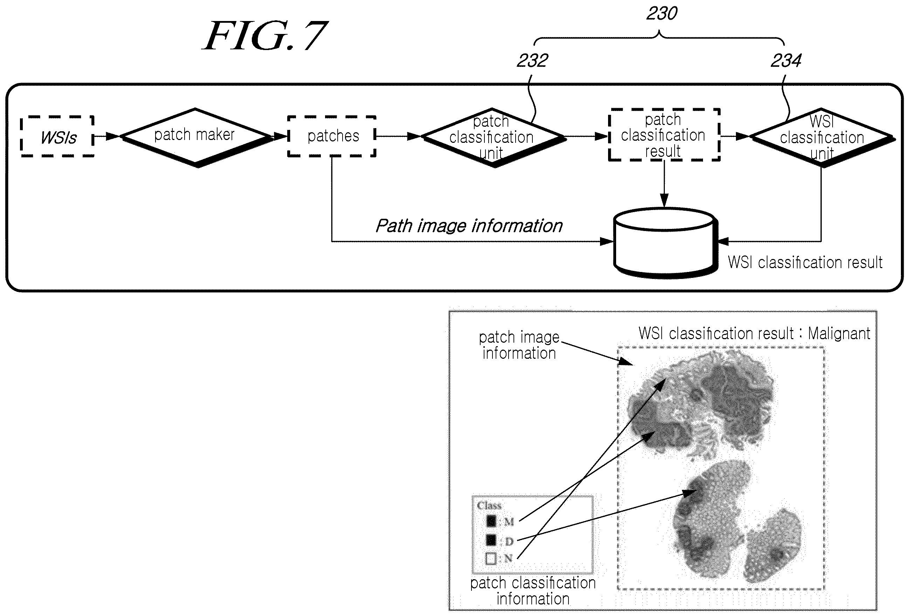

FIG. 7 illustrates an operation flow and the results of the tissue classification unit of FIG. 2 classifying a tissue using an artificial intelligence model.

In this step, the entire prediction framework is operated based on the model trained in the previous step to generate results and additional information. The prediction method may be configured to be similar to the training order mentioned above. For the developed models, the models for each of the gastric biopsy and colon biopsy are individually constructed and trained. Each model may be operated in the same order.

The operation process of the model of the trained slide tissue image classification unit is configured of tasks similar to the order of the training tasks.

If one new slide tissue image (WSI) is input to the tissue classification unit 230, the patch maker generates several patch images and patch information such as indexes and location information thereabout from the input slide tissue image and record them in the storage unit 240.

Each patch image is input to the patch classification unit 232 and a group is inferred by the model, generating a patch classification result, and storing it in the storage unit 240. Finally, the slide tissue image classification unit 234 generates a reconstructed slide tissue image by integrating patch image information and patch classification information of the storage unit 240 and classifies the slide tissue image.

The process includes converting into the reconstructed slide tissue image by integrating each patch classification information and location information, inputting it to the slide tissue image classification unit 234 to generate the slide tissue image classification result and storing it in the storage unit 240.

Therefore, three types of information are generated and stored in the storage unit 240, which may be patch image information, patch classification information, and slide tissue image classification information. The patch image information includes the patch index, the patch image, patch location information, or the like, and the patch classification information means the group inference result generated from the patch classification model, and the slide tissue image classification information means group information inferred by the slide tissue image classification model for one slide tissue image.

The image classification information for quality management purposes of routine practice is configured to comprehensively provide the three pieces of information mentioned above to the user. The patch image information provides visualization for the original image and location information about each patch in the user interface, providing visual information about the slide image in the user interface.

The patch classification information is used to provide classification information (heat map) about each patch on the slide image appearing in the user interface, which helps to provide a basis for classification results and lesion location information for quality control. The display unit 250 visually provides the location of the lesion based on the slide tissue image classification information about the storage unit 240 and the patch image prediction information.

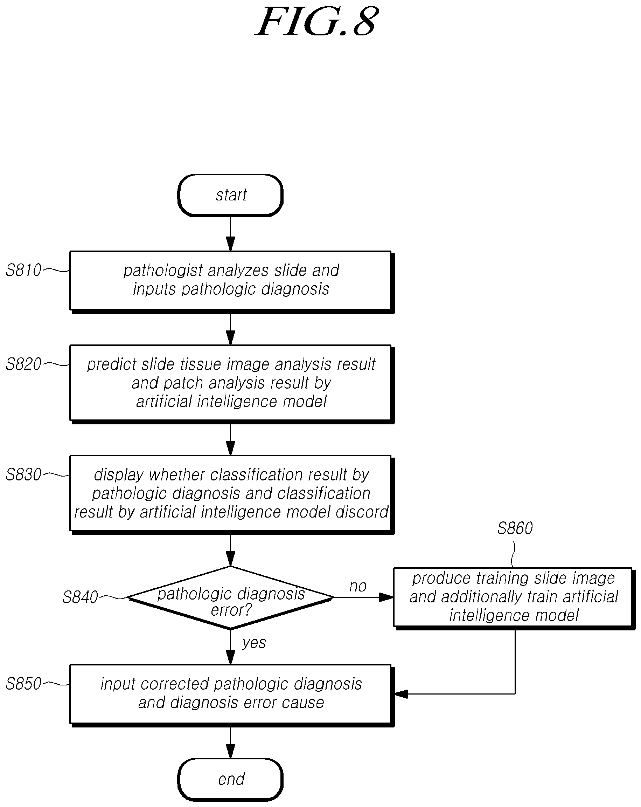

FIG. 8 is a flowchart illustrating slide reading results by a pathologist and slide tissue image classification results by an artificial intelligence model through the histopathologic reading support device of FIG. 2.

The histopathologic reading support device 200 of FIG. 2 supports a series of processes in which daily reading slides are scanned and converted into slide tissue images while artificial intelligence reading and visualization and information processing of the results are performed.

The histopathologic reading support device 200 may perform daily scanning and artificial intelligence prediction on all slides read after microscopic reading.

Referring to FIGS. 2 and 8, a pathologist performs reading under a microscope. Rather than being prepared separately according to specimens such as gastric biopsy and colon biopsy, slides are simply mixed without organ distinction in the order of reception number and provided to the pathologist. Therefore, the pathologist also read them in order of reception number without organ distinction. The input unit 210 inputs a slide diagnosis result obtained by reading a slide corresponding to the slide tissue image under a microscope or through a screen by a pathologist (S810).

For example, the input unit 210 is an input device such as a keyboard or a mouse, and may input the analyzed slide reading result typed by the pathologist or by mouse clicks.

The scanner 110 scans the read slides. The slide tissue image generated by the scanner 110 is a file with a specific extension, e.g., a file with the mrxs extension, and is stored in a path designated by the scanner program. If a new slide tissue image file is generated in the path, it is copied to the storage unit 250.

The histopathologic reading support device 200 searches the storage unit 240 for the scanned slide name (pathology number) and calls and drives one of two or more artificial intelligence models corresponding to each organ according to designated keywords (see Table 2). The artificial intelligence model reads the slide tissue image file and performs prediction on the patch classification result and the slide tissue image classification result, and stores each prediction result in the storage unit 240 (S820).

**[Table 2]**

| | |
|---|---|
| Al models | keywords |
| Gastric | Stomach, Esophagogastric junction, Gastroesophageal junction |
| Colorectal | Terminal ileum, Ileocecal valve, Cecum, Colon, Large intestine, Recto-sigmoid junction, Colon and Rectum, Rectum |

The display unit 250 may additionally display whether the slide tissue image classification result by the tissue classification unit 230 and the slide classification result by the pathologic diagnosis discord together with the slide pathologic diagnosis result by the pathologist (S830).

Specifically, the scanned slides are organized into a group by pathologic diagnosis and a group by artificial intelligence prediction, and pathologists may access the histopathologic reading support device 200 the next day (or hours later) to identify whether the groups accord and review discording cases in priority.

This allows the pathologist to "review" discording cases and, if necessary, "correct" the initial result (pathologic diagnosis error due to a human error).

The pathologist may determine whether there is a pathologic diagnosis error for the discording cases (S840), and if the pathologic diagnosis is incorrect among the discording cases, the pathologist may input the corrected pathologic diagnosis and the cause of the created diagnosis error using the input unit 210 (S850).

Conversely, when the slide tissue image classification result by the tissue classification unit 230 among the discording cases is incorrect, as described above, a training slide tissue image with an annotation included in the corresponding to slide tissue image is created, and the artificial intelligence model may be additionally trained (S860).

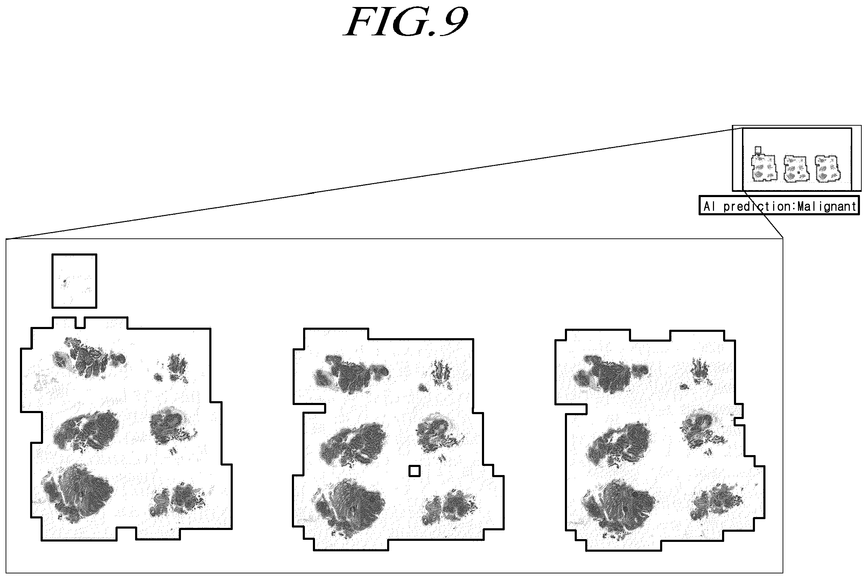

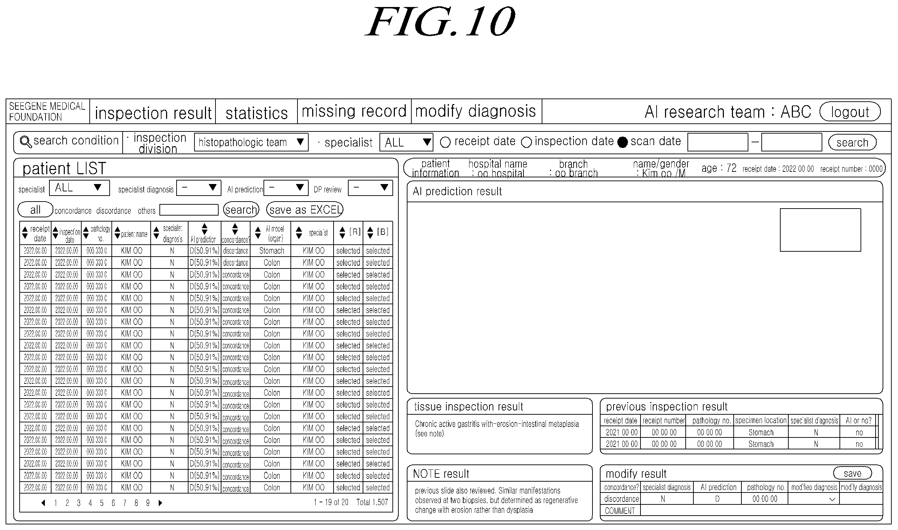

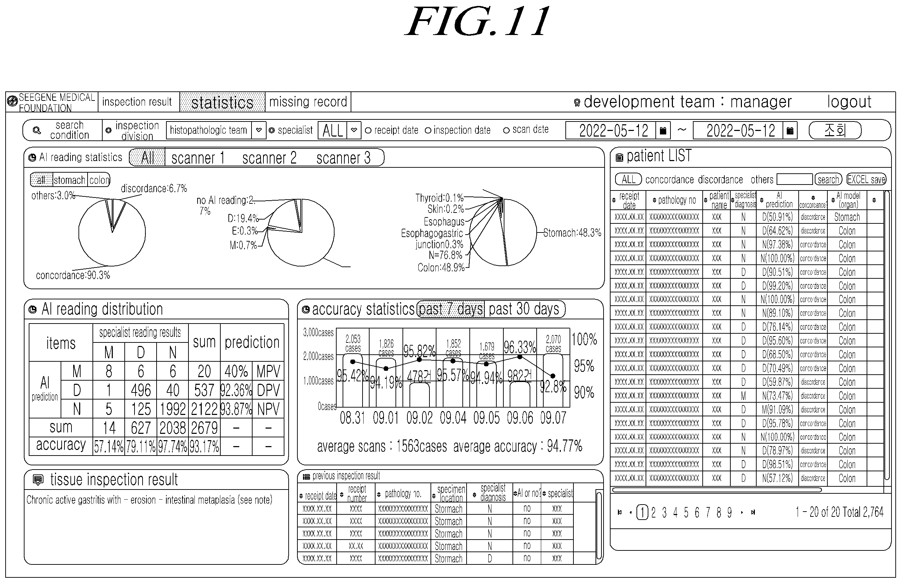

FIG. 9 illustrates a slide tissue image displayed on the display unit of FIG. 2. FIG. 10 illustrates an entire reading result, a slide preview image, an artificial intelligence reading result, and previous result information displayed on the display unit of FIG. 2. FIG. 11 illustrates statistical information and whether the slide reading result and the slide tissue image classification result discord, displayed on the display unit of FIG. 2.

As shown in FIG. 9, the heat map color for each patch displayed on the slide tissue image represents the result of the group inferred from the corresponding patch.

The histopathologic reading support device 200 may search for the scanned slide data for each receipt date, inspect date, and scan date through the inspection result/statistics page.

First, the inspection result page provides a slide information list for the receipt date for each slide, inspect data, pathology No. (slide name, patient name, classification by pathologic diagnosis, classification by AI prediction, concordance, AI model by anatomy, or pathologist reader).

Through the list, the corresponding to slide tissue image and heat map may be identified. Further, text information, such as AI model heat map thumbnail with a single magnification (0.5x) and pathologic diagnosis, notes, and previous pathological diagnosis, is provided. It is very difficult for the pathologist to review all read slide tissue images 100% for quality control purposes only while performing key tasks in daily life. Therefore, it is possible to perform a filtering function to separately search for only cases in which the classification result by pathologic diagnosis and the classification result by artificial intelligence prediction discord.

Further, if the pathologist reviews the slide tissue image he or she read, the corresponding row is highlighted to let it intuitively known whether review has been performed.

Further, on the statistics page, the predicted performance and distribution for each artificial intelligence model (gastric/colorectal) may be identified, and because the corresponding slide tissue images may be reviewed through a method of clicking on each cell in the table, the pathologists may selectively review cases meeting desired conditions even through the statistics page, as well as through the inspection result page.

If the scanned slide is searched and a specific slide row or column is double-clicked, the slide tissue image viewer for the corresponding slide shows up. The image position may be moved by dragging the screen, the mouse wheel may be operated to support the enlargement/reduction of the slide tissue image, and the position displayed on the current screen may be identified through a mini map at the upper right end.

Further, a list of associated slides is provided at the upper left end of the slide tissue image viewer so that related slide tissue images may be bundled and viewed if one specimen includes several slides (e.g., when there are recuts, serial or deeper sections, or when there are two or more blocks). Further, various slide tissue image-related functions may be used through a function button at the lower and left end.

Since artificial intelligence models are based on black box techniques, it is very difficult to determine which features of the data the model has learned and for what reason the prediction results were derived. Therefore, it is difficult to unconditionally trust the results of artificial intelligence predictions, and in fact, it requires an appropriate basis for judgment of the results and verification of their validity and reliability. Accordingly, there is provided a function of visualizing to together grasp the slide tissue image prediction information and the patch prediction information on a single slide tissue image on the slide tissue image viewer.

Referring back to FIG. 9, the slide tissue image prediction information is displayed as a text label at the lower end of the mini-map at the upper right end of the slide tissue image viewer. The patch prediction information is implemented in the form of a heat map, and prediction information is represented on each patch in the slide tissue image based on location information about each patch.

The location of the group M patch is red heat, the group D patch is blue heat, and the group N patch is no heat, and the color used for slide annotation when training the model may be used the same. In particular, it may be implemented so that the heat map is simply 'mask-on and -off' through right-click while viewing the slide through the slide tissue image viewer. The slide tissue image and the heat map may be allowed to be simultaneously viewed at all magnifications (0.5x to 40x) by the basic functions of the slide tissue image viewer, such as enlargement/reduction and rotation. Therefore, when reviewing the slide tissue image, the pathologist may intuitively make comparison as to which part and how the artificial intelligence model inferred each part through a single slide tissue image, while frequently masking on/off the heat map at all magnifications.

Research on the advancement of artificial intelligence classification models is still in progress, and continuous feedback from pathologists on the slide tissue image-level and patch-level performance of artificial intelligence models applied in practice is needed to measure and enhance the performance of artificial intelligence models. Therefore, it is possible to allow the heat map of the artificial intelligence model and the qualitative evaluation of the prediction to be recorded in each slide tissue image list. This evaluation information will be used as additional data to enhance model performance in the future.

According to the classifications in Table 2 above, if the corresponding keyword is included in the pathologic diagnosis (text information), it is classified into each group, and the classification results are compared 1:1 with the classification results according to the artificial intelligence model prediction, and becomes the reference for determining whether they accord or discord.

The priorities for applying classifications for each keyword may be M>U>D>N. However, "sessile serrated adenoma/polyp", "sessile serrated adenoma", "sessile serrated lesion" may be prioritized over the keywords of item D.

For example, "Adeno***carcinoma*,** moderately differentiated""Neoplastic lesion, suspicious for ***malignancy"*,** *"adenoma, **high*** grade *dysplasia" "Tubulovillous adenoma, low* to focal ***high*** grade *dysplasia*" "with focal ***carcinoma***tous change""*adenoma,* ***grade uncertain", "Malignan*t** neoplasm", "Atypical glandular proliferation, ***favor neoplastic",** "Neuroendocrine **carcinoma"*** or such diagnosis include 'keywords in italics' and are finally classified by 'keywords in bold'. Tubular ***adenoma, low*** grade ***dysplasia",*** "Atypical glandular proliferation, favor ***dysplasia",*** "glandular proliferation, ***indefinite for dysplasia",*** "glandular proliferation, ***undetermined significance"*** or such diagnosis are classified as D according to the above rule. "Small cell nests with ***neuroendocrine*** feature" is also classified as U together with ***"Neuroendocrine*** tumor, grade 1 **(*carcinoid*** tumor)".

According to the classification rule, when "Tubulovillous *adenoma*, *low* to focal ***high*** grade *dysplasia"* is classified as "M" by the pathologic diagnosis, but is classified as "D" by the slide tissue image artificial intelligence prediction, so that they are different from each other, the case may be indicated as "discordance", which is to be reexamined by the pathologist. For ambiguous diagnosis cases such as "atypical glandular proliferation, ***favor neoplastic",*** "glandular proliferation, ***undetermined significance",*** "glandular proliferation, ***indefinite for dysplasia",*** their pathologic diagnosis-based classifications are indicated as "M, D, and D", respectively, but may be predicted as M, D, or N according to the manifestation of each case by the slide tissue image prediction. If these atypical cases are also indicated as "Discordance" with the prediction by the artificial intelligence, they may be re-examined by the pathologist.

Further, in such atypical cases, serial cut or recut slides are often produced, in which case the artificial intelligence prediction results may be different for each slide tissue image. For example, there may be a case where the prediction for the original slide is D, one of the serial cuts is D, and another is M, and the last deeper cut, as all the legions disappear, is N. As such, when the artificial intelligence predictions of the consecutive slides produced as one block differ from each other, the final prediction may be marked in the order of M>U>D>N.

In the above example, the artificial intelligence prediction may finally be marked as "M". If the pathologic diagnosis is "TA, LGD", it will be marked as "D", therefore, this may also be considered a "discordance" case. If the goal is simply to enhance the concordance rate (accuracy) between diagnosis by the specialist and artificial intelligence prediction, it is enough to make a setting so that it is finally classified as D which is the most among D, D, M, and N according to the prediction results of the slides of each cut. However, for the purpose of post-analytic quality control and to quickly detect false negatives, clinically more critical results may be prioritized over the most weighted artificial intelligence prediction results.

Because it is composed of such a system, in fact, the accuracy of artificial intelligence predictions cannot but be lower than that of other artificial intelligence models for diagnostic assistance purposes. However, it is not efficient to review more cases than necessary, so it is necessary to secure an ideal level of accuracy that may perform both efficient and practical functions.

The histopathologic reading support device 200 according to an embodiment may develop an artificial intelligence model with reliable performance that may reduce pathologists' daily potential human errors, especially false negatives while maintaining the optical microscope-based workflow without infringing upon or threatening the pathologists' responsibility for diagnosis-not diagnosing better than pathologists-and may be applied as the most realistic and suitable method for the routine practice of gastrointestinal endoscopic biopsy reading as "a tool of daily fast QC".

Further, the histopathologic reading support device 200 according to an embodiment may prevent patients in need of treatment from being missed to effectively detect false negatives due to potential human errors in routine work (practice), and take necessary measures immediately.

Further, the histopathologic reading support device 200 according to an embodiment may reflect artifacts and variables of the field to be directly applied to routine practice.

Further, the histopathologic reading support device 200 according to an embodiment does not aim to provide criteria for accurate screening of 'invasive cancer and dysplasia', 'tumorous change and reactive change', which are subject to the inherent authority of each pathologist and respected.

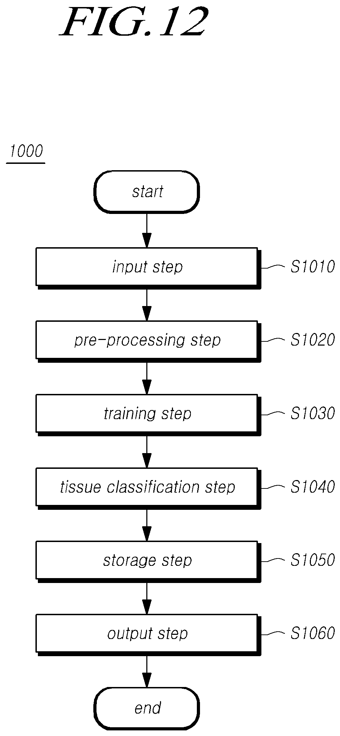

FIG. 12 is a flowchart illustrating a histopathologic reading support method according to another embodiment.

Referring to FIG. 12, a histopathologic reading support method 1000 includes an input step S1010, a pre-processing step S1020, a training step S1030, a tissue classification step S1040, a storage step S1050, and an output step S1060.

The input step S1010 inputs slide information and a slide tissue image.

As described with reference to FIG. 4, the pre-processing step S1020 divides the input slide tissue image into a plurality of patches.

As described with FIGS. 5 and 7, when there is a legion, the training step S1030 performs training an artificial intelligence model with patch classification results by dividing a training slide tissue image marked with a site of the legion into a plurality of patches and inferring a classification of each of the plurality of patches and training the artificial intelligence model with a slide tissue classification result by inferring a classification of a slide tissue image reconstructed by integrating the plurality of patches and the classification results of the patches;

As described with FIG. 7, the tissue classification step S1040 generates a patch classification result by inferring a classification of each of the plurality of patches using a trained artificial intelligence model, and generates a slide tissue classification result by inferring a classification of a slide tissue image reconstructed by integrating the plurality of patches and the classification results of the patches.

The storage step S1040 stores the slide information and the slide tissue image, the plurality of patches, patch information about the patches, the patch classification result, and the slide tissue classification result.

The output step S1050 outputs the patch classification result of each of the plurality of patches and the slide tissue classification result.

When there is the legion, the training slide tissue image may indicate the site of the legion by a single closed curve in a different color for each legion.

The training slide tissue image may use the slide tissue image scanned by a slide scanner, as it is, without any artificial enhancement.

There are two or more trained artificial intelligence models, and the slide information includes an organ of the slide tissue image, and in the tissue classification step S1030, the tissue image may be classified by selecting one of two or more artificial intelligence models according to the organ of the slide tissue image. As described above, as the trained artificial intelligence model, artificial intelligence models trained for the organs of the stomach and colon have been described as an example. However, in addition to the illustrated stomach and colon models, models for other organs (breasts, prostate, skin, etc.) may be further developed in a similar way and applied as a routine quality control system in the same way.

As described with reference to FIGS. 7 and 9, in the output step S1060, the patch classification result may be displayed in a unique color for each location of the lesion of each patch, and the slide tissue classification result may be displayed as one of a letter, a number, and a color.

As described with reference to FIG. 7, the reconstructed slide tissue image is reconstructed by combining the plurality of patches displayed in a unique color for each location of the lesion, and the output step S1060 may display the slide tissue analysis results indicated by letters or numbers together with the reconstructed slide tissue image.

The input step S1010 inputs a pathologic diagnosis result obtained by reading a slide corresponding to the slide tissue image under a microscope or through a screen by a pathologist.

Later, in the display step S1060, as described with reference to FIG. 10, in the tissue classification step S1040, whether the slide tissue image classification result and the classification result by the pathologic diagnosis discord may be additionally displayed together with the pathologic diagnosis result.

Specifically, what is input in the input step S1010 may be content analyzed and read by the pathologist, i.e., "pathologic diagnosis" information (text information). This pathologic diagnosis information may be "classified" as one of three groups M, D, and N by a classification rule. The artificial intelligence model may independently predict one of the three groups M, D, and N. The display step S1060 displays whether "classification result by pathologic diagnosis information (text information)" and "classification result by artificial intelligence model (image information)" corresponding thereto accord or discord.

By additionally displaying whether the slide tissue image classification result and the classification result by pathologic diagnosis discord in the tissue classification step S1040, it is possible to manage the diagnostic quality of the routine practice by establishing an automatic verification system for diagnosis of the artificial intelligence model in the histopathologic reading routine practice.

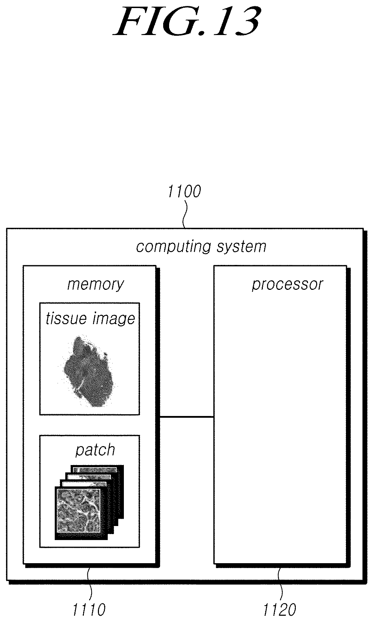

FIG. 13 is a block diagram illustrating a computing system 1100 according to embodiments of the present invention.

Referring to FIG. 13, a computing system 1100 may include a memory 1110 and a processor 1120.

The memory 1110 may store the slide tissue image where the tissues have been captured and the plurality of patches into which the slide tissue image is divided, or may separately store them in, e.g., a separate high-capacity storage server. The memory 1110 may be a volatile memory (e.g., SRAM or DRAM) or nonvolatile memory (e.g., NAND Flash).

When there is a legion, the processor 1120 may perform training an artificial intelligence model with patch classification results by dividing a training slide tissue image marked with a site of the legion into a plurality of patches and inferring a classification of each of the plurality of patches and training the artificial intelligence model with a slide tissue classification result by inferring a classification of a slide tissue image reconstructed by integrating the plurality of patches and the classification results of the patches; The processor 1120 may generate a patch classification result by inferring a classification of each of the plurality of patches using a trained artificial intelligence model, and generate a slide tissue classification result by inferring a classification of a slide tissue image reconstructed by integrating the plurality of patches and the classification results of the patches.

The histopathologic reading support device 200 may be configured as the computing system 1100 illustrated in FIG. 13, or may be configured as a GPU server including storage for storing the scan file (WSI image), a GPU processor, and a general memory, but the disclosure is not limited thereto.

The above-described histopathologic reading support device 200 may be implemented by a computing device including at least some of a processor, a memory, a user input device, and a presentation device. The memory is a medium that stores computer-readable software, applications, program modules, routines, instructions, and/or data, coded to perform specific tasks when executed by a processor. The processor may read and execute the computer-readable software, applications, program modules, routines, instructions, and/or data stored in the memory. The user input device may be a means for allowing the user to input a command to the processor to execute a specific task or to input data required for the execution of the specific task. The user input device may include a physical or virtual keyboard or keypad, key button, mouse, joystick, trackball, touch-sensitive input means, or a microphone. The presentation device may include, e.g., a display, a printer, a speaker, or a vibrator.

The computing device may include various devices, such as smartphones, tablets, laptops, desktops, servers, clients, and the like. The computing device may be a single stand-alone device and may include a plurality of computing devices operating in a distributed environment composed of a plurality of computing devices cooperating with each other through a communication network.

Meanwhile, the computing device may be a quantum computing device rather than a classical computing device. The quantum computing device performs operations in qubit units rather than bits. The qubit may have a state in which 0 and 1 are superposed at the same time, and if there are M qubits, 2^M states may be expressed at the same time.

The quantum computing device may use various types of quantum gates (e.g., Pauli/Rotation/Hadamard/CNOT/SWAP/Toffoli) that receive one or more qubits to perform a quantum operation and performs a designated operation and may configure a quantum circuit with a special function by combining the quantum gates.

The quantum computing device may use a quantum artificial neural network (e.g., QCNN or QGRNN) that may perform the functions performed by the conventional artificial neural network (e.g., CNN or RNN), using fewer parameters at higher speed.

Further, the above-described histopathologic reading support method 1000 may be executed by a computing device that includes a processor and a memory storing computer readable software, applications, program modules, routines, instructions, and/or data structures, coded to perform the above-described histopathologic reading support method 1000 when executed by the processor.

The present embodiments described above may be implemented through various means. For example, the present embodiments may be implemented by various means, e.g., hardware, firmware, software, or a combination thereof.

When implemented in hardware, the histopathologic reading support method 1000 according to the present embodiments may be implemented by, e.g., one or more application specific integrated circuits (ASICs), digital signal processors (DSPs), digital signal processing devices (DSPDs), programmable logic devices (PLDs), field programmable gate arrays (FPGAs), processors, controllers, micro-controllers, or micro-processors.

For example, the histopathologic reading support method 1000 according to embodiments may be implemented by an artificial intelligence semiconductor device in which neurons and synapses of the deep neural network are implemented with semiconductor devices. In this case, the semiconductor devices may be currently available semiconductor devices, e.g., SRAM, DRAM, or NAND or may be next-generation semiconductor devices, such as RRAM, STT MRAM, or PRAM, or may be combinations thereof.

When the histopathologic reading support method 1000 according to embodiments is implemented using an artificial intelligence semiconductor device, the results (weights) of training the deep learning model with software may be transferred to synaptic mimic devices disposed in an array structure, or learning may be performed in the artificial intelligence semiconductor device.

When implemented in firmware or hardware, the histopathologic reading support method 1000 according to the present embodiments may be implemented in the form of a device, procedure, or function performing the above-described functions or operations. The software code may be stored in a memory unit and driven by a processor. The memory unit may be positioned inside or outside the processor to exchange data with the processor by various known means.

The above-described terms, such as "system," "processor," "controller," "component," "module," "interface," "model," or "unit," described above may generally refer to computer-related entity hardware, a combination of hardware and software, software, or software being executed. For example, the above-described components may be, but are not limited to, processes driven by a processor, processors, controllers, control processors, entities, execution threads, programs, and/or computers. For example, both an application being executed by a controller or a processor and the controller or the processor may be the components. One or more components may reside within a process and/or thread of execution, and the components may be positioned in one device (e.g., a system, a computing device, etc.) or distributed in two or more devices.

Meanwhile, another embodiment provides a computer program stored in a computer recording medium for performing the above-described histopathologic reading support method 1000. Further, another embodiment provides a computer-readable recording medium storing a program for realizing the above-described histopathologic reading support method.

The program recorded on the recording medium may be read, installed, and executed by a computer to execute the above-described steps.

As such, for the computer to read the program recorded on the recording medium and execute the implemented functions with the program, the above-described program may include code coded in a computer language, such as C, C++, JAVA, or machine language, which the processor (CPU) of the computer may read through a computer device interface.

Such code may include a function code related to a function defining the above-described functions or may include an execution procedure-related control code necessary for the processor of the computer to execute the above-described functions according to a predetermined procedure.

Further, the code may further include additional information necessary for the processor of the computer to execute the above-described functions or memory reference-related code as to the position (or address) in the internal or external memory of the computer the media should reference.

Further, when the processor of the computer needs to communicate with, e.g., another computer or a server at a remote site to execute the above-described functions, the code may further include communication-related code as to how the processor of the computer should communicate with the remote computer or server using the communication module of the computer and what information or media should be transmitted/received upon communication.

The above-described computer-readable recording medium may include, e.g., ROMs, RAMs, CD-ROMs, magnetic tapes, floppy disks, or optical data storage devices, or may also include carrier wave-type implementations (e.g., transmissions through the Internet).

Further, the computer-readable recording medium may be distributed to computer systems connected via a network, and computer-readable codes may be stored and executed in a distributed manner.

The functional programs for implementing the present invention and code and code segments related thereto may easily be inferred or changed by programmers of the technical field to which the present invention pertains, considering, e.g., the system environments of the computer reading and executing the program.

The histopathologic reading support method 1000 described in connection with FIG. 12 may be implemented in the form of recording media including computer-executable instructions, such as application or program modules. The computer-readable medium may be an available medium that is accessible by a computer. The computer-readable storage medium may include a volatile medium, a non-volatile medium, a separable medium, and/or an inseparable medium. The computer-readable medium may include a computer storage medium. The computer storage medium may include a volatile medium, a non-volatile medium, a separable medium, and/or an inseparable medium that is implemented in any method or scheme to store computer-readable commands, data architecture, program modules, or other data or information.

The above-described histopathologic reading support method 1000 may be executed by an application installed on a terminal, including a platform equipped in the terminal or a program included in the operating system of the terminal), or may be executed by an application (or program) installed by the user on a master terminal via an application providing server, such as a web server, associated with the service or method, an application, or an application store server. In such a sense, the above-described histopathologic reading support method 1000 may be implemented in an application or program installed as default on the terminal or installed directly by the user and may be recorded in a recording medium or storage medium readable by a terminal or computer.

Although embodiments of the present invention have been described with reference to the accompanying drawings, It will be appreciated by one of ordinary skill in the art that the present disclosure may be implemented in other various specific forms without changing the essence or technical spirit of the present disclosure. Thus, it should be noted that the above-described embodiments are provided as examples and should not be interpreted as limiting. Each of the components may be separated into two or more units or modules to perform its function(s) or operation(s), and two or more of the components may be integrated into a single unit or module to perform their functions or operations.

It should be noted that the scope of the present invention is defined by the appended claims rather than the described description of the embodiments and include all modifications or changes made to the claims or equivalents of the claims.

## Claims

1. A histopathologic reading support device, comprising:
an input unit inputting slide information and a slide tissue image;
a pre-processing unit dividing the input slide tissue image into a plurality of patches;
a tissue classification unit generating a patch classification result by inferring a classification of each of the plurality of patches using a trained artificial intelligence model, and generating a slide tissue classification result by inferring a classification of a slide tissue image reconstructed by integrating the plurality of patches and the classification results of the patches;
a storage unit storing the slide information and the slide tissue image, the plurality of patches, patch information about the patches, the patch classification result, and the slide tissue image classification result; and
an output unit outputting the patch classification result of each of the plurality of patches and the slide tissue image classification result,
wherein when there is a legion, the trained artificial intelligence model is trained with patch classification results by dividing a training slide tissue image marked with a site of the legion into a plurality of patches using the pre-processing unit and inferring a classification of each of the plurality of patches and is trained with a slide tissue image classification result by inferring a classification of a slide tissue image reconstructed by integrating the plurality of patches and the classification results of the patches.

2. The histopathologic reading support device of claim 1, wherein when there is the legion, the training slide tissue image indicates the site of the legion by a single closed curve in a different color for each legion.

3. The histopathologic reading support device of claim 1, wherein the training slide tissue image uses a slide tissue image scanned by a slide scanner, as it is, without artificial enhancement.

4. The histopathologic reading support device of claim 1, wherein there are provided two or more trained artificial intelligence models, wherein the slide information includes an organ of the slide tissue image, and
wherein the tissue classification unit classifies a tissue image by selecting one of the two or more artificial intelligence models according to the organ of the slide tissue image.

5. The histopathologic reading support device of claim 1, wherein the output unit indicates the patch classification result in a unique color for each legion position of each patch, and indicates the slide tissue image classification result with at least one of a letter, a number, or a color.

6. The histopathologic reading support device of claim 5, wherein the reconstructed slide tissue image is reconstructed by combining a plurality of patches indicated in unique colors for each legion position, and
wherein the output unit displays the slide tissue image analysis result indicated with the letter or the number together with the reconstructed slide tissue image.

7. The histopathologic reading support device of claim 1, wherein the input unit inputs a pathologic diagnosis which is a slide analysis result of reading a slide corresponding to the slide tissue image through a microscope or the slide tissue image through a screen by a pathologist, and
wherein the display unit additionally displays a concordance or a discordance between the slide tissue image classification result by the tissue image classification unit and the classification result by the pathologic diagnosis together with the pathologic diagnosis which is the slide reading result by the pathologist.

8. The histopathologic reading support device of claim 1, wherein when the classification result by the pathologic diagnosis is incorrect when there is a discordance between the slide tissue image classification result and the classification result by the pathologic diagnosis, a corrected pathologic diagnosis and a cause of a created diagnosis error are input using the input unit.

9. The histopathologic reading support device of claim 8, wherein when there is the discordance between the slide tissue image classification result and the classification result by the pathologic diagnosis, if the slide tissue image classification result is incorrect, the training slide tissue image is created with an annotation included in the corresponding to slide tissue image, and the artificial intelligence model is additionally trained.

10. The histopathologic reading support device of claim 1, wherein when several slides are present in one specimen, the output unit provides an associated slide list at an upper left end of a slide tissue image viewer so that related slide tissue images are bundled and viewed.

11. A histopathologic reading support method, comprising:
an input step inputting slide information and a slide tissue image;
a pre-processing step dividing the input slide tissue image into a plurality of patches;
when there is a legion, a training step training an artificial intelligence model with patch classification results by dividing a training slide tissue image marked with a site of the legion into a plurality of patches and inferring a classification of each of the plurality of patches and training the artificial intelligence model with a slide tissue image classification result by inferring a classification of a slide tissue image reconstructed by integrating the plurality of patches and the classification results of the patches;
a tissue classification step generating a patch classification result by inferring a classification of each of the plurality of patches using a trained artificial intelligence model, and generating a slide tissue image classification result by inferring a classification of a slide tissue image reconstructed by integrating the plurality of patches and the classification results of the patches;
a storage step storing the slide information and the slide tissue image, the plurality of patches, patch information about the patches, the patch classification result, and the slide tissue image classification result; and
an output step outputting the patch classification result of each of the plurality of patches and the slide tissue image classification result.

12. The histopathologic reading support method of claim 11, wherein when there is the legion, the training slide tissue image indicates the site of the legion by a single closed curve in a different color for each legion.

13. The histopathologic reading support method of claim 11, wherein the training slide tissue image uses a slide tissue image scanned by a slide scanner, as it is, without artificial enhancement.

14. The histopathologic reading support method of claim 11, wherein there are provided two or more trained artificial intelligence models, wherein the slide information includes an organ of the slide tissue image, and
wherein the tissue image classification step classifies a tissue image by selecting one of the two or more artificial intelligence models according to the organ of the slide tissue image.

15. The histopathologic reading support method of claim 11, wherein the output step indicates the patch classification result in a unique color for each legion position of each patch, and indicates the slide tissue image classification result with one of a letter, a number, or a color.

16. The histopathologic reading support method of claim 15, wherein the reconstructed slide tissue image is reconstructed by combining a plurality of patches indicated in unique colors for each legion position, and
wherein the output step displays the slide tissue image classification (or inference) result indicated with the letter or the number together with the reconstructed slide tissue image.

17. The histopathologic reading support method of claim 16, wherein the input step inputs a pathologic diagnosis which is a slide reading result of analyzing a slide corresponding to the slide tissue image through a microscope or the slide tissue image through a screen by a pathologist, and
wherein the display step additionally displays a concordance or a discordance between the slide tissue image classification result by the tissue image classification step and the classification result by the pathologic diagnosis together with the pathologic diagnosis which is the slide reading result by the pathologist.

18. The histopathologic reading support method of claim 11, wherein when the classification result by the pathologic diagnosis is incorrect when there is a discordance between the slide tissue image classification result and the classification result by the pathologic diagnosis, a corrected pathologic diagnosis and a cause of a created diagnosis error are input by the input step.

19. The histopathologic reading support method of claim 18, wherein when there is the discordance between the slide tissue image classification result and the classification result by the pathologic diagnosis, if the slide tissue image classification result is incorrect, the training slide tissue image is created with an annotation included in the corresponding to slide tissue image, and the artificial intelligence model is additionally trained.

20. The histopathologic reading support method of claim 11, wherein when several slides are present in one specimen, the output step provides an associated slide list at an upper left end of a slide tissue image viewer so that related slide tissue images are bundled and viewed.
